# EUROPÄISCHE PATENTANMELDUNG

(11) **EP 1 180 379 A1**
(43) Veröffentlichungstag der Anmeldung: **20.02.2002**
(21) Anmeldenummer: 01118586.5
(22) Anmeldetag: 02.08.2001
(51) Int. Cl.: A61M 25/00

(54) **Doppellumiger Katheter mit Kontrollelement**

(30) Priorität: 17.08.2000 DE 10040350
(71) Anmelder: REHAU AG + Co, 95111 Rehau (DE)
(72) Erfinder: Weisser, Norbert, 95111 Rehau (DE)

(57) **Zusammenfassung**

Die Erfindung betrifft einen Katheter für die Behandlung von Fluiden in Ventrikeln und/oder Subarachnoidalräumen sowie dem Hirngewebe, der einfach handhabbar und wirtschaftlich herstellbar ist und bei dem gleichzeitig mit der Zuführung und dem Abfluss von Fluiden eine Überwachung des intracraniellen Druckes kontinuierlich und jederzeit möglich ist. Es soll weiterhin eine optische Kontrolle der zuzuführenden bzw. abfließenden Fluide möglich sein und die Lage des Katheters jederzeit mit der geeigneten Technik lokalisieren zu können, ohne dass es zu Artefakten oder Auslöschen von Hirnstrukturen aufgrund von Überlagerungen durch das Kathetermaterial kommt.
Erfindungsgemäß wird dies dadurch gelöst, dass der Katheter (1) aus einem polymeren Werkstoff besteht, wobei dieser wenigstens zwei Lumen (2,3) aufweist, die miteinander verbunden sind, wobei in jedes Lumen, beginnend am freien Ende (10) des Katheters (1), wenigstens eine Öffnung (21,31) eingebracht ist und wobei am freien Ende (10) des Katheters (1) ein Kontrollelement (4) eingebracht ist.

## Beschreibung

In der neurochirurgischen, neurologischen, neonatologischen und pädiatrischen Therapie ist der Einsatz von Kathetern für die Behandlung von Fluiden in Ventrikeln und/oder Subarachnoidalräumen bzw. des Hirngewebes bei einer Vielzahl von Erkrankungen mit Hirndrucksymptomatik durch eine externe Liquordrainage bekannt. Diese bekannten Einmalsysteme sind nicht für eine ständige Überwachung des Liquordruckes über einen Monitor bei gleichzeitiger Drainage ausgelegt.
Für eine ständige Drucküberwachung und Drainage sind Mikrochipkatheter oder fieberoptische Katheter bekannt, deren Anschaffung sehr kostenintensiv ist und die mit einer aufwendigen Messtechnik versehen demzufolge nur bei besonderen Erkrankungen eingesetzt werden.
Die erforderliche und notwendige Messung des intracraniellen Druckes erfolgt mittels eines externen Druckwandlersystemes.

Bei diesen Kathetern aus dem bekannten Stand der Technik ist es nachteilig, dass eine gleichzeitige Druckmessung und Drainage nicht möglich ist.
Ein weiterer Nachteil besteht darin, dass bei der Anwendung im Bereich des menschlichen Gehirns der Katheter mittels geeigneter technischer Maßnahmen lokalisierbar sein muss. Dies ist bisher nur mit solchen Kathetern möglich, die durch den Einsatz bestimmter chemischer Stoffe voll konstrastiert sind.
Die Vollkontrastrierung führt zu einer rauen Oberflächenstruktur der Katheter mit den Nachteilen der Anlagerung von Proteinen, Bakterien und Zellbestandteilen.
Weiterhin ist eine optische Kontrolle der Fluiden durch die Vollkontrastierung der Katheter nicht möglich.

Die Katheter werden derzeit so verwendet, dass diese mittels spezieller und aufwendiger Verbindungselemente den erforderlichen Funktionen Druckmessung und Drainage nacheinander zugeführt werden.

Die damit verbundenen Probleme und Schwierigkeiten, insbesondere die Diskonnektionsmöglichkeit mit der Gefahr der Bakterieninfektion des Gehirnes stellt einen schwerwiegenden und nicht akzeptierbaren Nachteil dar. Die medizinisch geforderte kontinuierliche Aufzeichnung des intracraniellen Druckes wird durch die Drainagezyklen unterbrochen.

Die Erfindung hat sich nun die Aufgabe gestellt, die Nachteile des bekannten Standes der Technik zu vermeiden und einen Katheter aufzuzeigen, der einfach handhabbar und wirtschaftlich herstellbar ist und bei dem gleichzeitig mit der Zuführung und dem Abfluss von Fluiden eine Überwachung des intracraniellen Druckes kontinuierlich und jederzeit möglich ist.
Es soll weiterhin eine optische Kontrolle der zuzuführenden bzw. abfließenden Fluide möglich sein und die Lage des Katheters jederzeit mit der geeigneten Technik lokalisieren zu können, ohne dass es zu Artefakten oder Auslöschen von Hirnstrukturen auf Grund von Überlagerungen durch das Kathetermaterial kommt.

Erfindungsgemäß wird dies dadurch gelöst, dass der Katheter aus einem polymeren Werkstoff besteht, wobei dieser wenigstens zwei Lumen aufweist, die miteinander verbunden sind, wobei in jedes Lumen, beginnend am freien Ende des Katheters, wenigstens eine Öffnung eingebracht ist und wobei am freien Ende des Katheters ein Kontrollelement eingebracht ist.

Weitere vorteilhafte Ausgestaltungen sind den Unteransprüchen zu entnehmen.

Vorteilhaft wird gesehen, dass der erfindungsgemäße Katheter aus einem transparenten polymeren Werkstoff besteht, durch den jederzeit eine optische Kontrolle der zugeführten bzw der abfließenden Fluide durch das medizinische Personal möglich ist.

Ein entscheidender Vorteil des erfindungsgemäßen Katheters besteht darin, dass bei der erforderlichen Durchführung neuer Therapieformen bei cerebralen Blutungen die gleichzeitige Liquordrainage und die kontinuierliche Druckmessung erstmals möglich sind.
Ein weiterer Vorteil wird darin gesehen, dass es für den Patienten zu keiner zusätzlichen Traumatisierung des Gehirns kommt, da bei der herkömmlichen Behandlung von Fluiden in den Ventrikeln und/oder Subarachnoidalräumen und/oder des Gehirns immer zwei Trepanationslöcher in der Schädeldecke erforderlich sind, die durch die Verbindung mehrerer Funktionen im erfindungsgemäßen Katheter nicht mehr erforderlich sind.
Ein weiterer Vorteil wird darin gesehen, dass durch den erfindungsgemäßen Katheter dessen Positionierung in den durch cerebrale Blutungen befallenen Ventrikeln und/oder Subarachnoidalräumen durch das Kontrollelement sehr schnell und genau möglich ist.

Der erfindungsgemäße Katheter hat eine hohe Anwendersicherheit, da durch diesen keine Konnektion auf herkömmliche Katheterverbindungselemente erforderlich ist und damit auch mögliche Leckagen auszuschließen sind.
Aufgrund des geometrisch einfachen Aufbaues und der verwendeten Materialien ist der erfindungsgemäße Katheter kostengünstig herstellbar und trotzdem leicht handhabbar.

Im Rahmen der Erfindung liegt auch die Verwendung eines Katheters mit beispielsweise drei Lumen, wobei hier vorteilhafterweise über ein Lumen der Zufluss von Medikamenten in die mit cerebralen Blutungen versehenen Ventrikel und/oder Subarachnoidalräume zugeführt wird, mit dem Ziel, die vorhandenen Hämatome zu verflüssigen, wobei das zweite Lumen dem Abfluss der verflüssigten Hämatome dient und einem dritten Lumen, durch das die kontinuierliche Druckmessung während dieser Behandlung ermöglicht ist.

Die Erfindung soll nun an einem dieser nicht beschränkenden Ausführungsbeispielen näher beschrieben werden. Es zeigt:

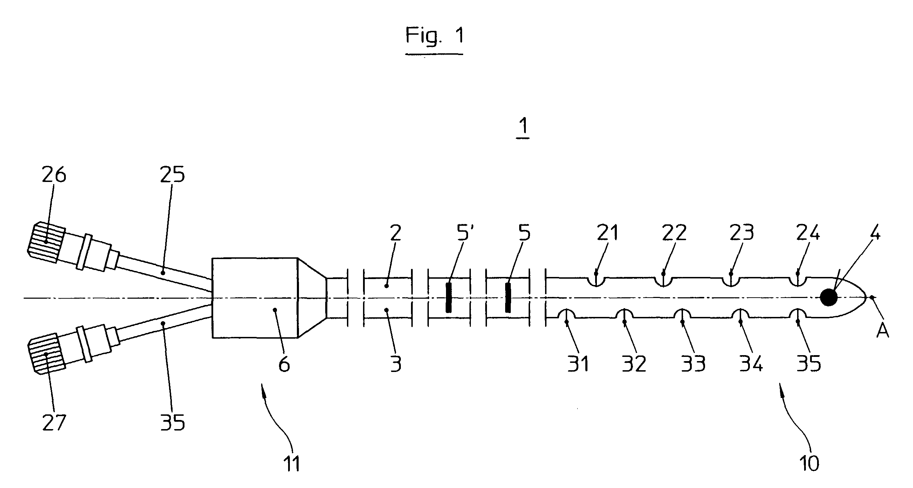

Figur 1: Katheter gemäß der Erfindung

In Figur 1 ist der erfindungsgemäße Katheter (1) dargestellt, der die Lumen (2, 3) aufweist und aus einem transparenten polymeren Werkstoff hergestellt ist.

Am freien Ende (10) des Katheters (1) ist das Kontrollelement (4) eingebracht, welches aus einem im Röntgen- und/oder Computertomograhie- und /oder MRT Magnetresonanztomographieverfahren sichtbaren Material besteht, beispielsweise aus Titan. Dieses Kontrollelement (4) ist in diesem Ausführungsbeispiel unmittelbar an der Spitze des freien Endes (10) des Katheters (1) auf der Längsachse (A) eingebracht und durch die Lumen (2, 3) vollflächig umschlossen.

Der Katheter (1) verfügt am freien Ende über in das Lumen (2, 3) eingebrachte Öffnungen (21, 22, 23, 24 und 31, 32, 33, 34, 35), die in diesem Ausführungsbeispiel sowohl diametral gegenüberliegend als auch alternierend gegenüberliegend eingebracht sind.
Der Abstand der Öffnungen (21, 22, 23, 24 und 31, 32, 33, 34, 35) beträgt in diesem Ausführungsbeispiel in etwa das Dreifache ihrer Abmessungen, wobei sowohl der Abstand als auch die Dimensionierung der Öffnungen (21, 22, 23, 24 und 31, 32, 33, 34, 35) vom jeweiligen Anwendungszweck abhängen und entsprechend den Anforderungen an die zu behandelnden Ventrikel bzw. Subarachnoidalräume anpassbar sind. Die Lumen (2, 3) des Katheters (1) sind in diesem Ausführungsbeispiel einstückig miteinander verbunden und aus dem gleichen transparenten polymeren Werkstoff hergestellt.
Es liegt jedoch auch im Rahmen der Erfindung, dass der Katheter (1) Lumen (2, 3) aufweist, die aus unterschiedlichen Werkstoffen hergestellt sind. Dies kann beispielsweise ein härteres Material für das Lumen (2) sein, welches stabilisierend bei der Einführung des Katheters (1) in die zu behandelnden Ventrikel und/oder Subarachnoidalräume dient und ein weicheres Material für das Lumen (3), welches sich der Geometrie in den Ventrikeln bzw. Subarachnoidalräumen anpasst.

Der Katheter (1) weist in diesem Ausführungsbeispiel, beginnend ab der Öffnung (31), Markierungselemente (5,5') auf, die beispielsweise in einer metrischen Skalierung aufgebracht sein können und dem medizinischen Personal eine zusätzliche Information beim Einführen des Katheters in die Ventrikel und/oder Subarachnoidalräume liefern.

Am freien Ende (11) des Katheters (1) ist ein Verbindungselement (6) dargestellt, in welchem die Lumen (2, 3) so fixiert werden, dass sie über die Schläuche (25, 35) zu den Anschlusselementen (26, 27) weiterleitbar sind, an die die entsprechenden Druckmessvorrichtungen bzw. Flüssigkeitszugabeelemente anschließbar sind. Das Verbindungselement (6) verhindert hier ein mögliches Trennen der Lumen (2, 3).

## Patentansprüche

1. Katheter (1) für die Behandlung von Fluiden in Ventrikeln und/oder Subarachnoidalräumen sowie dem Hirngewebe, bestehend aus polymerem Werkstoff, wobei der Katheter (1) wenigstens zwei Lumen (2, 3) aufweist, die miteinander verbunden sind, wobei in jedes Lumen (2, 3), beginnend am freien Ende (10) des Katheters (1), wenigstens eine Öffnung (21, 31) eingebracht ist und wobei am freien Ende der Vorrichtung (1) ein Kontrollelement (4) ist.

2. Katheter nach Anspruch 1, **dadurch gekennzeichnet, dass** der polymere Werkstoff transparent ist.

3. Katheter nach einem der vorhergehenden Ansprüche, **dadurch gekennzeichnet, dass** weitere Öffnungen (22, 23, 24 und 32, 33, 34, 35) axial in die Lumen (2, 3) eingebracht sind.

4. Katheter nach einem der vorhergehenden Ansprüche, **dadurch gekennzeichnet, dass** weitere Öffnungen (22, 23, 24 und 32, 33, 34, 35) radial in die Lumen (2, 3) eingebracht sind.

5. Katheter nach einem der vorhergehenden Ansprüche, **dadurch gekennzeichnet, dass** weitere Öffnungen (22, 23, 24 und 32, 33, 34, 35) der Lumen (2, 3) diametral gegenüberliegend angeordnet sind.

6. Katheter nach einem der vorhergehenden Ansprüche, **dadurch gekennzeichnet, dass** weitere Öffnungen (22, 23, 24 und 32, 33, 34, 35) der Lumen (2, 3) alternierend gegenüberliegend angeordnet sind.

7. Katheter nach einem der vorhergehenden Ansprüche, **dadurch gekennzeichnet, dass** weitere Öffnungen (22, 23, 24 und 32, 33, 34, 35) der Lumen (2, 3) einen Abstand zueinander aufweisen, der wenigstens ihren äußeren Abmessungen entspricht.

8. Katheter nach einem der vorhergehenden Ansprüche, **dadurch gekennzeichnet, dass** das Kontrollelement (4) aus einem im Röntgenverfahren sichtbaren Material besteht.

9. Katheter nach einem der vorhergehenden Ansprüche, **dadurch gekennzeichnet, dass** der Katheter, beginnend am freien Ende (10), Markierungselemente (5, 5') aufweist.
